# EUROPEAN PATENT APPLICATION

(11) **EP 2 399 917 A1**
(43) Date of publication of application: **28.12.2011**
(21) Application number: 10743591.9
(22) Date of filing: 22.02.2010
(51) Int. Cl.: C07D 413/14, A61K 31/513, A61P 3/00, A61P 3/04, A61P 3/10, A61P 9/00, A61P 9/10, A61P 9/12, A61P 13/12, A61P 25/00, A61P 27/02, A61P 29/00, A61P 43/00

(54) **NOVEL COMPOUNDS COMPRISING A 3-(5-ALKOXYPYRIMIDIN-2-YL) PYRIMIDIN-4(3H)-ONE STRUCTURE AND DRUGS THAT COMPRISE SAME**

(30) Priority: 23.02.2009 JP 2009039781
(71) Applicant: Kowa Company, Ltd., Nagoya-shi, Aichi-ken 460-8625 (JP)
(72) Inventor: MIURA, Toru, Higashimurayama-shi Tokyo 189-0022 (JP); ONOGI, Kazuhiro, Higashimurayama-shi Tokyo 189-0022 (JP); TAGASHIRA, Junya, Higashimurayama-shi Tokyo 189-0022 (JP); WATANABE, Gen, Higashimurayama-shi Tokyo 189-0022 (JP); SEKIMOTO, Ryohei, Higashimurayama-shi Tokyo 189-0022 (JP); ISHIDA, Rie, Higashimurayama-shi Tokyo 189-0022 (JP); OHGIYA, Tadaaki, Higashimurayama-shi Tokyo 189-0022 (JP)
(74) Representative: Albrecht, Thomas
(86) International application number: PCT/JP2010/001116
(87) International publication number: WO 2010/095462

(57) **Abstract**

Provided are novel compounds that have both angiotensin II receptor-antagonist effects and PPARy-activating effects that are useful as agents for preventing and/or treating hypertension, heart disease, angina, cerebral vascular accident, cerebrovascular disorder, ischemic peripheral circulatory disorder, kidney disease, atherosclerosis, inflammatory disease, type 2 diabetes mellitus, diabetic complication, insulin resistance syndrome, Syndrome X, metabolic syndrome, and hyperinsulinemia. Disclosed are 3-(5-alkoxypyrimidin--2-yl) pyrimidin-4(3H)-one derivatives represented by the General formula (I) : [wherein R represents a C₁₋₄ alkyl group], or salts or solvates thereof (I)

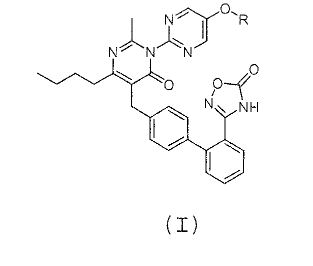

## Description

### TECHNICAL FIELD

The present invention relates to a novel compound having a structure of 3-(5-alkoxypyrimidin-2-yl)pyrimidin-4(3H)-one that have both angiotensin II antagonist activity and PPARγ activating activity, and a drug containing the same.

### BACKGROUND ART

In recent years, diseases such as diabetes, hypertension, dyslipidemia and obesity which can be a risk factor for arteriosclerotic diseases have been rapidly increasing due to changes in life style with improvements in living standard, i.e., high calorie and high cholesterol type diet, obesity, lack of exercise, aging, and the like. It is known that, although being a risk factor independent of each other, overlap of the diseases can cause an occurrence of arteriosclerotic diseases at higher frequency or aggravation of the diseases. As such, with the understanding of a condition having a plurality of risk factors for arteriosclerotic diseases as metabolic syndrome, efforts have been made to elucidate the cause of the syndrome and to develop a therapeutic method therefor.

Angiotensin II (AII) is a peptide that is found to be an intrinsic pressor substance produced by renin-angiotensin system (i.e., RA system) . It is believed that pharmacological inhibition of AII activity can lead to treatment or prevention of circulatory diseases such as hypertension. Accordingly, an inhibitor for angiotensin converting enzyme (ACE) which inhibits the enzyme promoting the conversion of angiotensin I (AI) to angiotensin II (AII) has been clinically used as an inhibitory agent for RA system. Furthermore, an orally administrable AII receptor blocker (Angiotensin Receptor Blocker: ARB) has been developed, and losartan, candesartan, telmisartan, valsartan, olmesartan, irbesartan and the like are already clinically used as a hypotensive agent. It is reported by many clinical or basic studies that, as having not only a hypotensive activity but also other various activities including an anti-inflammatory activity, an endothelial function improving activity, a cardiovascular remodeling inhibiting activity, an oxidation stress inhibiting activity, a proliferation factor inhibiting activity, insulin resistance improving activity and the like, ARB is useful for cardiovascular diseases, renal diseases, arteriosclerosis, and the like. (Non-Patent Documents 1 and 2). Most recently, it is also reported that ARB particularly has a kidney protecting activity which does not depend on a hypotensive activity (Non-Patent Document 3).

Meanwhile, three isoforms, i.e., α, γ, and δ, have been identified so far for peroxisome proliferator-activated receptors (PPARs) which belong to a nuclear receptor superfamily. Among them, PPARγ is an isoform most abundantly expressed in an adipose tissue and it plays an important role in differentiation of adipocytes or metabolism of glycolipids. Currently, thiazolidinedione derivatives (i.e., TZD) such as pioglitazone or Rosiglitazone are clinically used as a therapeutic agent for diabetes having PPARγ activating activity, and they are known to have an activity of improving insulin resistance, glucose tolerance, lipid metabolism, and the like. Further, it is recently reported that, based on activation of PPARγ, TZD exhibits various activities including a hypotensive activity, an anti-inflammatory activity, an endothelial function improving activity, a proliferation factor inhibiting activity, an activity of interfering RA system, and the like. It is also reported that, according to such multiple activities, TZD shows a kidney protecting activity particularly in diabetic nephropathy without depending on blood sugar control (Non-Patent Documents 4, 5, 6, 7, and 8). Meanwhile, there is also a concern regarding adverse effects of TZD caused by PPARγ activation, such as body fluid accumulation, body weight gain, peripheral edema, and pulmonary edema (Non-Patent Documents 9 and 10).

It has been recently reported that telmisartan has a PEARγ activating activity (Non-Patent Document 11). It has been also reported that the irbesartan has the same activity (Non-Patent Document 12) . These compounds have both an RA system inhibiting activity and a PPARγ activating activity, and thus are expected to be used as an integrated agent for prevention and/or treatment of circulatory diseases (e.g., hypertension, heart disease, angina pectoris, cerebral vascular accident, cerebrovascular disorder, ischemic peripheral circulatory disorder, kidney disease, and the like.) or diabetes-related diseases (e.g., type 2 diabetes mellitus, diabetic complication, insulin resistant syndrome, metabolic syndrome, hyperinsulinemia, and the like.) without increasing a risk of body fluid accumulation, body weight gain, peripheral edema, pulmonary edema, or congestive heart failure that are concerned over the use of TZD (Patent Document 1). Among them, for diabetic nephropathy, a synergistic prophylactic and/or therapeutic effect is expected from composited kidney protecting activity based on activities of RA system inhibition and PPARγ activation.
As a compound having the activities above, the pyrimidine and triazine derivatives have been reported (Patent Document 1). In Patent Document 1, a compound having the pyrimidinone skeleton as the following formula (A) is disclosed (see, Example 325 of Patent Document 1):

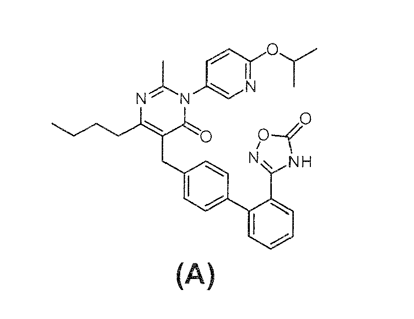

However, a compound having a 2-pyrimidyl group directly bonded to position 3 of the pyrimidinone ring is not specifically described in Patent Document 1.

### CITATION LIST

### PATENT DOCUMENT

Patent Document 1: WO 2008/062905

### NON-PATENT DOCUMENT

Non-Patent Document 1: AMER. J. Hypertension, 18, 720 (2005)
Non-Patent Document 2: Current Hypertension Report, 10, 261 (2008)
Non-Patent Document 3: Diabetes Care, 30, 1581 (2007)
Non-Patent Document 4: Kidney Int., 70, 1223 (2006)
Non-Patent Document 5: Circulation, 108, 2941 (2003)
Non-Patent Document 6: Best Pract. Res. Clin. Endocrinol. Metab., 21(4), 687 (2007)
Non-Patent Document 7: Diab. Vasc. Dis. Res., 1(2), 76 (2004)
Non-Patent Document 8: Diab. Vasc. Dis. Res., 2(2), 61 (2005)
Non-Patent Document 9: J. Clin. Invest., 116(3), 581 (2006)
Non-Patent Document 10: FASEB J., 20(8), 1203 (2006)
Non-Patent Document 11: Hypertension, 43, 993 (2004)
Non-Patent Document 12: Circulation, 109, 2054 (2004)

### SUMMARY OF THE INVENTION

### PROBLEMS TO BE SOLVED BY THE INVENTION

An object of the invention is to provide a novel compound that is useful as an agent for preventing and/or treating hypertension as a circulatory disease, diabete as a metabolic disease, and the like.

### MEANS FOR SOLVING THE PROBLEMS

As a result of intensive studies to achieve the purpose described above, the inventors of the invention found that the 3-(5-alkoxypyrimidin-2-yl)pyrimidin-4(3H)-one derivative represented by the formula (I) below has both an excellent AII antagonist activity and an excellent PPARγ activating activity, in particular an excellent AII receptor antagonist activity, and therefore completed the invention.

Specifically, the invention relates to the following inventions.
(1) A 3-(5-Alkoxypyrimidin-2-yl)pyrimidin-4(3H)-one derivative represented by the formula (I) below or a salt thereof, or a solvate thereof:

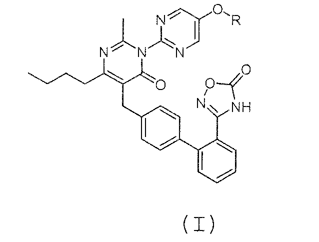

wherein R represents a C₁₋₄ alkyl group.
(2) The compound or the salt thereof, or the solvate thereof described in the above (1), wherein the C₁₋₄ alkyl group for R is a methyl group or an ethyl group.
(3) A pharmaceutical composition containing: the compound or the salt thereof, or the solvate thereof described in the above (1) or (2), and a pharmaceutically acceptable carrier.
(4) A pharmaceutical composition containig: the compound or the salt thereof, or the solvate thereof described in the above (1) or (2) as an effective component, having both an angiotensin II receptor antagonist activity and a PPARγ activating activity.
(5) An agent for preventing and/or treating a circulatory disease containing as an effective component the 3-(5-alkoxypyrimidin-2-yl)pyrimidin-4(3H)-one derivative or the salt thereof, or the solvate thereof described in the above (1) or (2).
(6) The agent for preventing and/or treating a circulatory disease described in the above (5), wherein the circulatory disease is hypertension, heart disease, angina pectoris, cerebral vascular accident, cerebrovascular disorder, ischemic peripheral circulatory disorder, kidney disease, or atherosclerosis.
(7) An agent for preventing and/or treating a metabolic disease containing as an effective component the 3-(5-alkoxypyrimidin-2-yl)pyrimidin-4(3H)-one derivative or the salt thereof, or the solvate thereof described in the above (1) or (2).
(8) The agent for preventing and/or treating a metabolic disease described in the above (7), wherein the metabolic disease is type 2 diabetes mellitus, diabetic complication (diabetic retinopathy, diabetic neuropathy, or diabetic nephropathy), insulin resistant syndrome, metabolic syndrome, or hyperinsulinemia.
(9) A method of preventing and/or treating a circulatory disease **characterized in that** an effective amount of the 3-(5-alkoxypyrimidin-2-yl)pyrimidin-4(3H)-one derivative or the salt thereof, or the solvate thereof described in the above (1) or (2) is administered to a patient in need of the treatment.
(10) A method of preventing and/or treating a metabolic disease **characterized in that** an effective amount of the 3-(5-alkoxypyrimidin-2-yl)pyrimidin-4(3H)-one derivative or the salt thereof, or the solvate thereof described in the above (1) or (2) is administered to a patient in need of the treatment.
(11) Use of the 3-(5-alkoxypyrimidin-2-yl)pyrimidin-4(3H)-one derivative or the salt thereof, or the solvate thereof described in the above (1) or (2) for production of a preparation used for prevention and/or treatment of a circulatory disease.
(12) Use of the 3-(5-alkoxypyrimidin-2-yl)pyrimidin-4(3H)-one derivative or the salt thereof, or the solvate thereof described in the above (1) or (2) for production of a preparation used for prevention and/or treatment of a metabolic disease.
(13) The 3-(5-alkoxypyrimidin-2-yl)pyrimidin-4(3H)-one derivative or the salt thereof, or the solvate thereof described in the above (1) or (2) having both an angiotensin II receptor antagonist activity and a PPARγ activating activity.

### EFFECTS OF THE INVENTION

The 3-(5-alkoxypyrimidin-2-yl)pyrimidin-4(3H)-one derivative represented by the formula (I) of the invention or the salt thereof, or the solvate thereof exhibits a potent antagonistic activity for an angiotensin II receptor, and can be appropriately used as an effective component for an agent for preventing and/or treating a disease related with angiotensin II, for example a circulatory disease such as hypertension, heart disease, angina pectoris, cerebral vascular accident, cerebrovascular disorder, ischemic peripheral circulatory disorder, kidney disease, and atherosclerosis.

Further, the 3-(5-alkoxypyrimidin-2-yl)pyrimidin-4(3H)-one derivative represented by the formula (I) of the invention or the salt thereof, or the solvate thereof has a PPARγ activating activity and can be appropriately used as an effective component for an agent for preventing and/or treating a disease related with PPARγ, for example a metabolic disease such as atherosclerosis, type 2 diabetes mellitus, diabetic complication (diabetic retinopathy, diabetic neuropathy, or diabetic nephropathy), insulin resistance syndrome, Syndrome X, metabolic syndrome, and hyperinsulinemia.

Still further, the 3-(5-alkoxypyrimidin--2-yl)pyrimidin-4(3H)-one derivative represented by the formula (I) of the invention, or the salt thereof, or the solvate thereof has both an antagonistic activity for an angiotensin II receptor and a PPARγ activating activity and can be appropriately used as an effective component for an agent for preventing and/or treating a disease related with both angiotensin II and PEARγ, for example atherosclerosis, diabetic nephropathy, insulin resistance syndrome, Syndrome X, and metabolic syndrome.

### BRIEF DESCRIPTION OF THE DRAWINGS

FIG. 1 illustrates inhibitory effect of each of the Compound a and the Comparative compound B on hypertensive activity of AII, wherein a male SD rat is used.
FIG. 2 illustrates a hypotensive activity of the Compound a, wherein a spontaneously hypertensive rat (SHR) is used.

### MODES FOR CARRYING OUT THE INVENTION

In the formula (I), examples of the C₁₋₄ alkyl group for R include a methyl group, an ethyl group, an n-propyl group, an isopropyl group, an n-butyl group, an isobutyl group, a sec-butyl group, and a tert-butyl group. The methyl group and ethyl group are preferable.

A salt of the compound represented by the formula (I) is not specifically limited, if it is a pharmaceutically acceptable salt. When the compound is treated as an acidic compound, examples include a salt with a metal salt such as sodium, potassium, magnesium and calcium, and the like. When the compound is treated as a basic compound, examples include mineral acid addition salts such as hydrochloric acid, hydrobromic acid, hydroiodic acid, sulfuric acid, nitric acid, and phosphoric acid salts; and organic acid addition salts such as methanesuulfonic acid, ethanesulfonic acid, benzenesulfonic acid, andp-toluenesulfonic acid salts, and the like.

Examples of the solvate of the compound represented by the formula (I) or a salt thereof include, for example, hydrates and the like, but the solvate is not limited to these examples.

In addition, a compound which are metabolized in a living body and converted into the compounds represented by the aforementioned formula (I), so called a prodrug, all fall within the scope of the compound of the invention. Examples of the group which forms the prodrug of the compound of the invention include the groups described in "Progress in Medicine," Vol. 5, pp. 2157-2161, 1985, Life Science Medica, and the groups described in "Development of Drugs, "Vol. 7, Molecular Designs, pp. 163-198, 1990, Hirokawa Shoten.

A compound represented by the formula (I), or a salt thereof or a solvate thereof can be produced according to the method described in the Examples herein later or other various known methods. For example, by using and cyclizing a 2-amino--5-substituted pyrimidine derivative, a derivative having a structure of 3-(5-substituted pyrimidin-2-yl)pyrlmidin-4(3H)-one structure can be produced. Further, for introducing the moiety R of the compound represented by the formula (I), position 5 of the pyrimidine ring is prepared to have a free hydroxy group and general alkylation for a phenolic hydroxy group can be carried out as shown in Process 4 of Example 1 described below. Further, when each process is performed, functional groups other than the reaction sites may be protected beforehand as required, and deprotected in an appropriate stage. Furthermore, each reaction may be performed by an ordinarily used method in each step, and isolation and purification can be performed by a method suitably selected from conventional methods such as crystallization, recrystallization, or chromatography, or a combination thereof.

Examples of dosage form of the medicament containing the compound of the invention, the salt thereof or the solvate thereof as an active component include, for example, those for oral administration such as tablet, capsule, granule, powder or syrup, and those for parenteral administration such as intravenous injection, intramuscular injection, suppository, inhalant, transdermal preparation, eye drop or nasal drop. In order to prepare medicinal formulations in the various dosage forms, the active component may be used alone, or may be used in appropriate combination with other pharmaceutically acceptable carriers such as excipients, binders, extending agents, disintegrating agents, surfactants, lubricants, dispersing agents, buffering agents, preservatives, corrigents, flavors, coating agents and diluents to obtain as a pharmaceutical composition.

Although the administration amount of the pharmaceutical agent of the invention may vary depending on the weight, age, sex, symptoms and the like of a patient, generally 0.1 to 1000 mg, especially 1 to 300 mg, as the compound represented by the formula (I), may be administered orally or parenterally at one time or several times as divided portions per day for an adult.

### EXAMPLES

Herein below, the invention will be explained in greater detail with reference to Examples. However, the invention is not limited to these Examples. The abbreviations used in the Examples have the following meanings.
s: singlet
d: doublet
t: triplet
q: quartet
m: multiplet
br: broad
J: coupling constant
Hz: Hertz
CDC1₃: deuterated chloroform
¹H-NMR: proton nuclear magnetic resonance

### Example 1

Production of 3-{4'-{[6-butyl-3-(5-ethoxypyrimidin-2-yl)-2-methylpyrimidi n-4(3H)-on-5-yl]methyl}biphenyl-2-yl}-1,2,4-oxadiazol-5(4H) -one (Compound a):

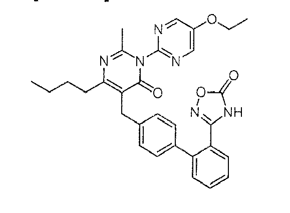

Process 1: Production of methyl (Z)-3-acetamide-2-[(2'-cyanobiphenyl-4-yl)methyl]-2-hepteno ate

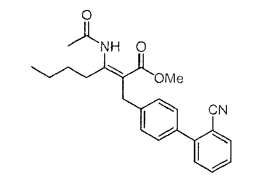

A mixture solution of toluene (40.5 mL) and acetic acid (4.5 mL) containing methyl 2-[(2'-cyanobiphenyl-4-yl)methyl]-3-oxoheptanoic acid (2.11 g, 6.04 mmol) and ammonium acetate (2.79 g, 36.2 mmol) were heated to reflux for 4 hours. The solvent was distilled off, and the resulting residue was added acetic anhydride (4.5 mL) and acetic acid (1.0 mL) at room temperature and stirred at 70°C for 3 hours. The reaction mixture was added water under ice cooling and extracted with ethyl acetate. The organic layer was combined, washed with water and brine, dried over anhydrous sodium sulfate, and concentrated in vacuo. The resulting residue was subjected to silica gel column chromatography (hexane : ethyl acetate = 4 : 1) to obtain 1.29 g of methyl (Z)-3-acetamide-2-[(2'-cyanobiphenyl-4-yl)methyl]-2-hepeten oate as a pale yellow oil (54.7%, 2 steps).

¹H-NMR (CDCl₃)δ:
0.89 (3H, t, J=7 Hz), 1.28-1.55 (4H, m), 2.17 (3H, s),
2.92 (2H, t, J=8 Hz), 3.70 (3H, s), 3.76 (2H, s),
7.25 (2H, d, J=8 Hz), 7.42 (1H, t, J=8 Hz),
7.45-7.53 (3H, m), 7.63 (1H, t, J=8 Hz),
7.74 (1H, d, J=8 Hz), 11.9 (1H, s).

Process 2: Production of 4'-{{3-[5-(benzyloxy)pyrimidin-2-yl]-6-butyl-2-methylpyrimi din-4(3H)-on-5-yl}methyl}biphenyl-2-carbonitrile

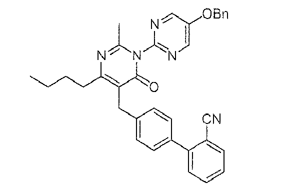

To a 1,2-dichloroethane (6 mL) solution of 2-amino-5-benzyloxypyrimidine (291 mg, 1.45 mmol), trimethyl aluminum (2 mol/L heptane solution, 1.21 mL, 2.42 mmol) was added at room temperature and stirred at the same temperature for 75 minutes. To the resulting solution, a 1,2-dichloroethane solution (4 mL) of methyl (Z)-3-acetamide-2-[(2'-cyanobiphenyl-4-yl)methyl]-2-hepeten oate (189 mg, 0.483 mmol) produced by process 1 was added dropwise at the same temperature, and heated to reflux for 12 hours. The reaction mixture was added an aqueous solution of ammonium chloride and chloroform, and filtered through a pad of celite. The organic layer was separated from the filtrate and the aqueous layer was extracted with chloroform. The organic layer was combined, washed with water and brine, dried over anhydrous sodium sulfate, and concentrated in vacuo. The resulting residue was subjected to silica gel column chromatography (hexane : acetone = 5 : 1 → 4 : 1) to obtain 236 mg of 4'-{{3-[5-(benzyloxy)pyrimidin-2-yl]-6-butyl-2-methylpyrimi din-4(3H)-on-5-yl}methyl}biphenyl-2-carbonitrile as a pale yellow amorphous (90.4%).

¹H-NMR (CDCl₃)δ:
0.92 (3H, t, d=7 Hz), 1.33-1.47 (2H, m), 1.51-1.68 (2H, m),
2.16 (3H, s), 2.65 (2H, t, J=8 Hz), 3.97 (2H, s),
5.22 (2H, s), 7.32-7.52 (11H, m), 7.61 (1H, t, J=8 Hz),
7.73 (1H, d, J=8 Hz), 8.60 (2H, s).

Process 3: Production of 4'-{[6-butyl-3-(5-hydroxypyrimidin-2-yl)-2-methylpyrimidin-4(3H)-on-5-yl]methyl}biphenyl-2-carbonitrile

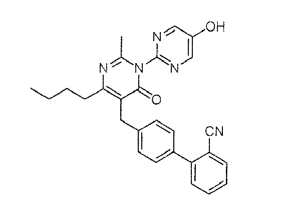

To a methanol (10 mL) solution of 4'-{{3-[5-(benzyloxy)pyrimidin-2-yl]-6-butyl-2-methylpyrimi din-4(3H)-on-5-yl}methyl}biphenyl-2-carbonitrile (235 mg, 0.434 mmol), 10% Pd-C (containing 50% moisture, 120 mg) was added for hydrogenation. After stirring for 1 hour at room temperature, the reaction mixture was filtered through a pad of celite, and washed with methanol. The residue obtained after distillation of the filtrate was subjected to silica gel column chromatography (chloroform : methanol = 10 : 1) to obtain 138 mg of 4'-{[6-butyl-3-(5-hydroxypyrimidin-2-yl)-2-methylpyrimidin-4(3H)-on-5-yl]methyl}biphenyl-2-carbonitrile (70.6%).

¹H-NMR (CDCl₃)δ:
0.93 (3H, t, d=7 Hz), 1.33-1.48 (2H, m), 1.53-1.67 (2H, m),
2.17 (3H, s), 2.69 (2H, t, J=8 Hz), 4.05 (2H, s),
7.32-7.46 (3H, m), 7.49 (2H, d,d=8 Hz), 7.62 (1H, t, J=8 Hz),
7.75 (1H, d, J=8 Hz), 8.16 (2H, s), 9.20 (1H, br s).

Process 4: Production of 4"-{[6-butyl-3-(5-ethoxypyrimidin-2-yl)-2-methylpyrimidin-4 (3H)-on-5-yl]methyl}biphenyl-2-carbonitrile

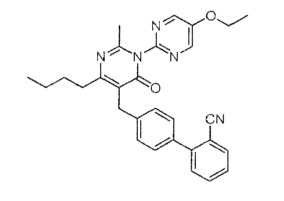

To an acetonitrile solution (1.5 mL) of 4'-{[6-butyl-3-(5-hydroxypyrimidin-2-yl)-2-methylpyrimidin-4(3H)-on-5-yl]methyl}biphenyl-2-carbonitrile (90 mg, 0.2 mmol), potassium carbonate (36 mg, 0.26 mmol) and iodoethane (37 mg, 0.24 mmol) were added and stirred at 65°C overnight. Upon the completion of the reaction, the reaction mixture was cooled to room temperature and filtered through a pad of celite by using ethyl acetate. The filtrate was concentrated in vacuo to obtain 120 mg of the crude product of 4'-{[6-butyl-3-(5-ethoxypyrimidin-2-yl)-2-methylpyrimidin-4 (3H)-on-5-yl]methyl}biphenyl-2-carbonitrile as a pale yellow oil.

¹H-NMR(CDCl₃) δ:
0. 92 (3H, t, J=7 Hz), 1.32-1.44 (2H, m), 1.50 (3H, t, J=7 Hz),
1.55-1.64 (2H, m), 2.16 (3H, s), 2.65 (2H, t, J=8 Hz),
3.98 (2H, s), 4.21 (2H, q, J=7 Hz), 7.35-7.49 (6H, m),
7.60 (1H, dt, J=2, 8 Hz), 7.73 (1H, d, J=8 Hz), 8.52 (2H, s) .

Process 5: Production of 4'-{[6-butyl-3-(5-ethoxypyrimidin-2-yl)-2-methylpyrimidin-4 (3H)-on-5-yl]methyl}-N'-hydroxybiphenyl-2-carboxyimidamide

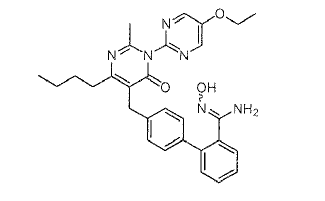

To a dimethyl sulfoxide solution (4 mL) of hydroxylamine hydrochloric acid salt (436 mg, 6.38 mmol), sodium hydrogen carbonate (633 mg, 7.54 mmol) was added and stirred at 40°C for 1 hour. To the reaction solution, a dimethyl sulfoxide solution (2 mL) of the crude product of 4'-{[6-butyl-3-(5-ethoxypyrimidin-2-yl)-2-methylpyrimidin-4 (3H)-on-5-yl]methyl}biphenyl-2-carbonitrile (120 mg) was added and stirred at 90°C overnight. The reaction mixture was added water and extracted with ethyl acetate. The organic layer was combined, washed with water and brine, dried over anhydrous sodium sulfate, and concentrated in vacuo. The resulting residue was subjected to silica gel column chromatography (ethyl acetate) to obtain 51 mg of 4'-{[6-butyl-3-(5-ethoxypyrimidin-2-yl)-2-methylpyrimidin-4 (3H)-on-5-yl]methyl}-N'-hydroxybiphenyl-2-carboxyimidamide as a pale yellow amorphous (49.8%, 2 steps yield).

¹H-NMR(CDCl₃) δ:
0. 91 (3H, t, J=7 Hz), 1.33-1.44 (2H, m), 1.50 (3H, t, J=7 Hz),
1.54-1.65 (2H, m), 2.15 (3H, s), 2.64 (2H, t, J=8 Hz),
3.94 (2H, s), 4.20 (2H, q, J=7 Hz), 4.40 (2H, br s),
7.29-7.49 (7H, m), 7.56 (1H, d, J=7.3 Hz), 8.51 (2H, s).

Process 6: Production of 3-{4'-{[6-butyl-3-(5-ethoxypyrimidin-2-yl)-2-methylpyrimidi n-4(3H)-on-5-yl]methyl}biphenyl-2-yl}-1,2,4-oxadiazol-5(4H) -one

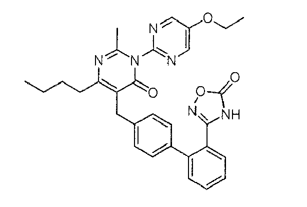

To a dimethyl formamide solution (1 mL) of 4'-{[6-butyl-3-(5-ethoxypyrimidin-2-yl)-2-methylpyrimidin-4 (3H)-on-5-yl]methyl}-N'-hydroxybiphenyl-2-carboxyimideamide (51 mg, 0.1 mmol), 1,1-carbonyldiimidazole (41 mg, 0.25 mmol) and 1,8-diazabicyclo[5.4.0]undec-7-ene (38 mg, 0.25 mmol) were added and stirred at room temperature for 1 hour. Upon the completion of the reaction, the reaction mixture was added water and extracted with ethyl acetate. The organic layer was combined, washed with water and brine, dried over anhydrous sodium sulfate, and concentrated in vacuo. The resulting residue was purified by silica gel column chromatography (ethyl acetate) to obtain 45 mg of 3-{4'-{[6-butyl-3-(5-ethoxypyrimidin-2-yl)-2-methylpyrimidi n-4(3H)-on-5-yl]methyl}biphenyl-2-yl}-1,2,4-oxadiazol-5(4H) -one (I) as a pale yellow amorphous (83.9%).

¹H-NMR (CDCl₃) δ:
0.93 (3H, t, J=7 Hz), 1.33-1.46 (2H, m), 1.49 (3H, t, J=7 Hz),
1.56-1.68 (2H, m), 2.12 (3H, s), 2.64 (2H, t, J=8 Hz),
3.88 (2H, s), 4.19 (2H, q, J=7 Hz), 7.17 (2H, d, J=8 Hz),
7.24-7.29 (2H, m), 7.34-7.46 (2H, m), 7.53-7.59 (1H, m),
7.73 (1H, d, J=8 Hz), 8.47 (2H, s).

### Example 2

Production of 3-{4'-{[6-butyl-3-(5-methoxypyrimidin-2-yl)-2-methylpyrimid in-4(3H)-on-5-yl]methyl}biphenyl-2-yl}-1,2,4-oxadiazol-5(4H )-one (Compound b):

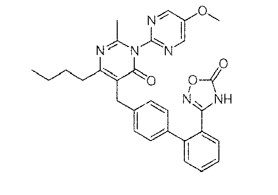

Process 1: Production of 4'-{[6-butyl-3-(5-methoxypyrimidin-2-yl)-2-methylpyrimidin-4(3H)-on-5-yl]methyl}biphenyl-2-carbonitrile

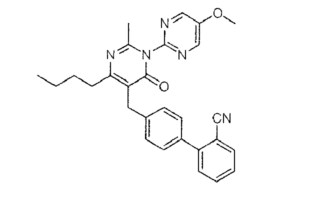

The title compound was obtained in the same reaction and treatment method as Process 2 of the Example 1 except that 2-amino-5-methoxypyrimidine was used instead of 2-amino-5-benzyloxypyrimidine.

¹H-NMR (CDCl₃) δ:
0,93 (3H, t, J=7 Hz), 1.33-1.46 (2H, m), 1.53-1.66 (2H, m),
2.16 (3H, s), 2.65 (2H, t, J=8 Hz), 3.98 (2H, s),
4.01 (3H, s), 7.34-7.49 (6H, m), 7.61 (1H, td, J=8, 1 Hz),
7.74 (1H, dd, J=8, 1 Hz), 8.54 (2H, s).

Process 2: Production of 4'-{[6-butyl-3-(5-methoxypyrimidin-2-yl)-2-methylpyrimidin-4(3H)-on-5-yl]methyl}-N'-hydroxybiphenyl-2-carboxyimidamide

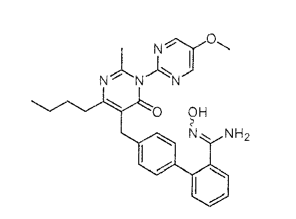

The title compound was obtained in the same reaction and treatment method as Process 5 of the Example 1 except that 4'-{[6-butyl-3-(5-methoxypyrimidin-2-yl)-2-methylpyrimidin-4(3H)-on-5-yl]methyl}biphenyl-2-carbonitrile was used instead of 4'-{[6-butyl-3-(5-ethoxypyrimidin-2-yl)-2-methylpyrimidin-4 (3H)-on-5-yl]methyl}biphenyl-2-aarbonitrile.

¹H-NMR (CDCl₃) δ:
0.92 (3H, t, J=7 Hz), 1.33-1.44 (2H, m), 1.53-1.63 (2H, m),
2.15 (3H, s), 2.65 (2H, t, J=8 Hz), 3.94 (2H, s),
4.00 (3H, s), 4.39 (2H, br s), 7.28-7.38 (6H, m),
7.44 (1H, t, J=8 Hz), 7.56 (1H, d, J=8 Hz), 8.54 (2H, s).

Process 3: Production of 3-{4'-{[6-butyl-3-(5-methoxypyrimidin-2-yl)-2-methylpyrimid in-4(3H)-on-5-yl]methyl}biphenyl-2-yl}-1,2,4-oxadiazol-5(4H )-one

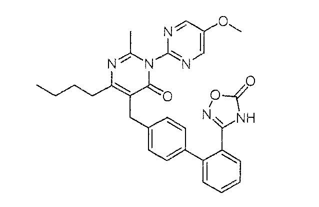

The title compound was obtained in the same reaction and treatment method as Process 6 of the Example 1 except that 4'-{[6-butyl-3-(5-methoxypyrimidin-2-yl)-2-methylpyrimidin-4(3H)-on-5-yl]methyl}-N'-hydroxybiphenyl-2-carboxyimidamide was used instead of 4'-{[6-butyl-3-(5-ethoxypyrimidin-2-yl)-2-methylpyrimidin-4 (3H)-on-5-yl]methyl}-N'-hydroxybiphenyl-2-carboxyimidamide.

¹H-NMR (CDCl₃) δ:
0.95 (3H, t, J=7 Hz), 1.37-1.48 (2H, m), 1.60-1.72 (2H,m),
2.15 (3H, s), 2.69 (2H, t, J=8 Hz), 3.91 (2H, s),
3.98 (3H, s), 7.21 (2H, d, J=8 Hz), 7.28 (2H, t, J=8 Hz),
7.38 (1H, d, J=8 Hz), 7.44 (1H, t, J=8 Hz),
7.57 (1H, t, J=8 Hz), 7.79 (1H, d, J=8Hz), 8.51 (2H, s).

### Test example 1: Angiotensin II antagonist activity in isolated rabbit blood vessels

By using a specimen of isolated rabbit blood vessels, antagonist activity of the Compound a or the Compound b against angiotensin II type 1 receptor was estimated from a dose-response curve of angiotensin II-induced blood vessel contraction. Specifically, the specimen of thoracic aorta ring of a rabbit (New Zealand White: male, 2.4 to 3.0 kg) was suspended in a magnus bath filled with Krebs-Henseleite buffer (composition: 118 mM NaCl, 4.7 mM KC1, 2.55 mM CaCl₂, 1.18 mM MgSO₄, 1.18 mM KH₂PO₄ 24.88 mM NaHCO₃, and 11.1 mM D-glucose), and angiotensin II (10 nM) -induced contraction was obtained in the presence of the Compound a or the Compound b (0.01 to 10 mol/L). During the measurement, the inside temperature of the magnus bath was maintained at 37°C and the bath was continuously ventilated with a sufficient amount of mixed gas (95% O₂ and 5% CO₂). The angiotensin II-induced contraction was converted into a relative value (%) that is based on the angiotensin II (10nM)-induced contraction in the absence of the Compound a or the Compound b. From the dose-response curve obtained therefrom, 50% inhibition concentration (IC₅₀ value) was calculated by using SAS Preclinical Package Ver 5. 0 (trade name, manufactured by SAS institute Japan Co., Tokyo, Japan), which is a statistical analysis program.

As a result of conducting the test described above, it was found that the Compound a and the Compound b have IC₅₀ value of 0.07 µM and 0.0075 µM, respectively, and thus the compound of the invention has a potent angiotensin II antagonist activity. Furthermore, with the test system herein, when the Compound A (i.e., compound of the Example 325 in Patent Document 1) and the Compound B (i.e., compound of the Example 47 in Patent Document 1) were used for the test under the same condition as above instead of the Compound a and the Compound b, it was found that the Compound A and the Compound B have IC₅₀ value of 0.37 µM and 0.022 µM, respectively.

### Test example 2: PPARγ activating activity

The agonist activity of the Compound a and the Compound b on PPARγ was measured based on the transfection assay using COS7 cells (DS Pharma Biomedical Co., Ltd., Osaka, Japan), which are the cell line derived from the kidney of the African green monkey. COS7 cells were cultured under 5% CO₂ concentration, and DMEM medium containing 10% fetal bovine serum, glutamic acid, and antibiotics was used as a medium.
As an expression vector, a chimera in which DNA binding domain of Ga14 which is a yeast transcription factor, and ligand binding domain of human PPARγ2 are fused, i.e., a fused product between the amino acids 1 to 147 of Ga14 transcription factor and the amino acids 182 to 505 of human PPARγ2, was used. Furthermore, as a reporter vector, a firefly luciferase containing five copies of Gal4 recognition sequence in the promoter region was used. Plasmid transfection of the cells was performed according to a method which uses jetPEI (trade name, manufactured by Funakoshi Co., Ltd., Tokyo, Japan). Furthermore, β-galactosidase expression vector was employed as an internal standard.
After the transfection of the cells, the medium was replaced with a DMEM medium (containing 1% serum) added the compound, and the cells were further cultured for 16 hours. After that, the luciferase activity and β-galaotosidase activity in the cell lysis solution were measured.
For the present test, dimethyl sulfoxide (DMSO) was used for dissolution and dilution of the Compound a or the Compound b, and during the cell treatment, the DMSO concentration in DMEM medium (containing 1% serum) was adjusted to 0.1%. As a positive compound, Rosiglitazone (trade name, manufactured by ALEXIS Corporation, Switzerland) was used. The percentage of the luciferase activity of the Compound a or the Compound b (1 to 30 µmol/L) was calculated when the luciferase activity of Rosiglitazone (3 to 10 µmol/L) is 100% and the luciferase activity in the absence of the Compound a or the Compound b is 0%. The 50% effective concentration of the compounds of the invention (EC₅₀, 50% effect concentration) was calculated by using SAS Preclinical Package Ver 5.0 (trade name, manufactured by SAS institute Japan Co., Tokyo, Japan), which is a statistical analysis program.

As a result of performing the test above, it was found that the Compound a and the Compound b have EC₅₀ value of 0.99 µM and 1.39 µM, respectively. The percentage of the luciferase activity, which had been calculated when the luciferase activity of Rosiglitazone is 100% and the luciferase activity in the absence of the Compound a or the Compound b is 0%, was 24% for the Compound b which has a methoxy group at position 5 of the pyrimidine ring. On the other hand, it was 51% for the Compound a which has an ethoxy group at position 5 of the pyrimidine ring. As such, it was confirmed that the compounds of the invention have a potent PPARγ activating activity. Furthermore, with the test system herein, when the Compound A (i.e., compound of the Example 325 in Patent Document 1) was tested instead of the Compound a or the Compound b under the same condition as above, it was found that the Compound A has EC₅₀ value of 0.67 µM and the percentage calculated from the luciferase activity was 52%.
Consequently, it was recognized that the compounds of the invention also have a sufficient PPARγ activating activity.

### Example 3

### Inhibitory activity on angiotensin II-induced blood pressure increase

The in vivo angiotensin II antagonist activity of the Compound a and the Compound B was evaluated by following, as an index, the blood pressure increase induced by intravenous injection of angiotensin II.
A male SD rat (10 to 12-week old) was anesthetized with pentobarbital, and a cannular for measuring blood pressure was inserted into the right femoral artery while a cannular for injecting angiotensin II was inserted into the right jugular vein, the cannulars were then fixed on the back side of the neck as exposed.
One day after the surgery, the rat was subjected to the test under unanaesthetized, unstrained, and unfasted condition. The artery cannular was connected to a blood pressure transducer, and the blood pressure and heart rate were continuously recorded on a polygraph with an aid of an amplifier. Once the blood pressure and heart rate are stabilized, angiotensin II (0.1 nmol/kg) was administered via the vein cannular. The angiotensin II was repeatedly administered until a stable hypertensive response is obtained. After that, the Compound a (1 mg/kg or 3 mg/kg) or the Compound B (3 mg/kg) was orally administered, and the hypertensive response induced by angiotensin II was observed over time until 24 hours after the administration. The blood pressure increase obtained was converted into a relative value (%) that is based on the blood pressure increase before the drug administration, yielding angiotensin II-induced blood pressure increase rate. Meanwhile, each of the Compound a and the Compound B was suspended in 0.5% methyl cellulose, and then used.

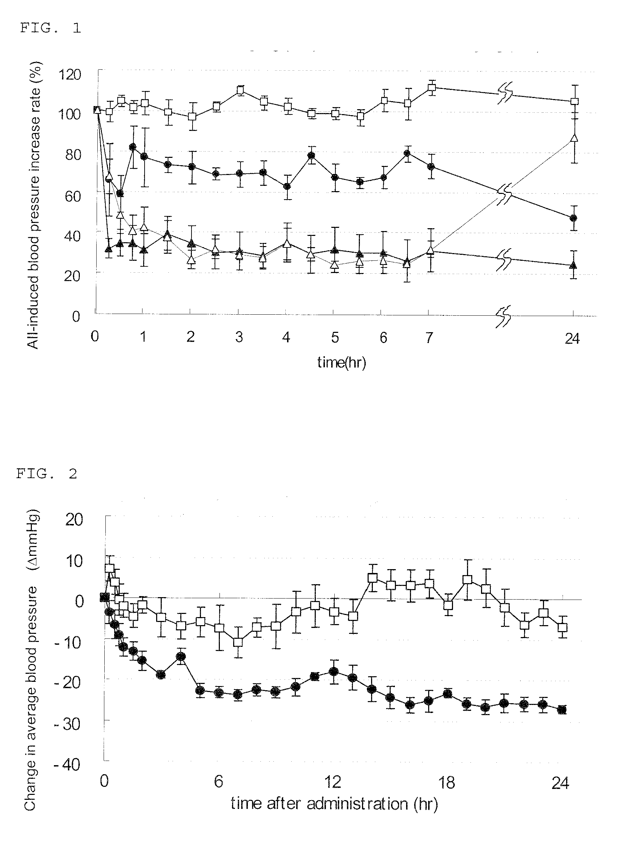

Results are shown in FIG. 1. The horizontal axis of FIG. 1 represents the time (hour) and the vertical axis represents the angiotensin II-induced blood pressure increase rate (%). In FIG. 1, an empty square (□) indicates a control group to which only the solvent (0.5% methyl cellulose) was administered (n = 5), a filled circle (•) indicates the administration of the Compound a (1 mg/kg, n=3), and a filled triangle (▲) indicates the administration of the Compound a (3 mg/kg, n = 6). Furthermore, an empty triangle (△) indicates the administration of the Compound B (3 mg/kg, n = 4). As a result, it was found that the Compound a shows a stable inhibitory action on angiotensin II-induced blood pressure increase even 24 hours after the administration, and thus a persistent and potent inhibitory activity of the Compound a on angiotensin II-induced blood pressure increase was confirmed. Meanwhile, although the administration of the Compound B with dosage of 3 mg/kg exhibited the effect of inhibiting the angiotensin II-induced blood pressure increase with substantially the same degree as the administration of the Compound a with dosage of 3 mg/kg, its effect was almost completely lost 24 hours after the administration.

### Example 4

### Hypotensive activity

Hypotensive activity of the Compound a was evaluated by following, as an index, the average blood pressure in spontaneously hypertensive rat (SHR) over time.
A SHR (17 to 22-week old) was anesthetized with pentobarbital, and a cannular for measuring blood pressure was inserted into the right femoral artery, and then fixed on the back side of the neck as exposed.
One day after the surgery, the SHR was subjected to the test under anaesthetized, unstrained, and unfasted condition. The artery cannular was connected to a blood pressure transducer, and the blood pressure and heart rate were continuously recorded on a polygraph with an aid of an amplifier. Once the blood pressure and heart rate are stabilized, the Compound a (3 mg/kg) was orally administered, and the average blood pressure was followed over time for next 24 hours. Meanwhile, 0.5% methyl cellulose was used as a solvent for the Compound a.

Results are shown in FIG. 2. The horizontal axis of FIG. 2 represents the time (hour) and the vertical axis represents the change in average blood pressure (ΔmmHg). In FIG. 2, an empty square (□) indicates a control group to which only the solvent (0.5% methyl cellulose) was administered (n = 7), and a filled circle (•) indicates the administration of the Compound a (3 mg/kg, n = 6) . As a result, it was found that the Compound a shows a stable hypotensive effect even 24 hours after the administration, and therefore the persistent and potent hypotensive activity of the Compound a was confirmed.

From the results shown above, it was confirmed that the Compound a has both a potent angiotensin II receptor antagonist activity and a PPARγ activating activity. It was also confirmed that the hypotensive activity based on the angiotensin II receptor antagonist activity is persistent. Therefore, it was found that the compound of the invention can be suitably used as an effective component for an agent for preventing and/or treating a disease related with angiotensin II and PPARγ, for example, hypertension, heart disease, angina pectoris, cerebral vascular accident, cerebrovascular disorder, ischemic peripheral circulatory disorder, kidney disease, atherosclerosis, inflammatory disease, type 2 diabetes mellitus, diabetic complication, insulin resistance syndrome, Syndrome X, metabolic syndrome, hyperinsulinemia, and the like.

### INDUSTRIAL APPLICABILITY

The compound of the formula (I) of the invention can be used as an effective component for an agent for preventing and/or treating a disease, for example, hypertension, heart disease, angina pectoris, cerebral vascular accident, cerebrovascular disorder, ischemic peripheral circulatory disorder, kidney disease, atherosclerosis, inflammatory disease, type 2 diabetes mellitus, diabetic complication, insulin resistance syndrome, Syndrome X, metabolic syndrome, hyperinsulinemia, and the like, and therefore the compound has an industrial applicability.

## Claims

1. A 3-(5-alkoxypyrimidin-2-yl)pyrimidin-4(3H)-one derivative represented by the formula (I) below or a salt thereof, or a solvate thereof: 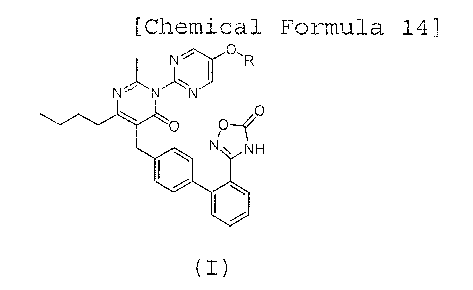 wherein R represents a C₁₋₄ alkyl group.

2. The compound or the salt thereof, or the solvate thereof according to Claim 1, wherein the C₁₋₄ alkyl group for R is a methyl group or an ethyl group.

3. A pharmaceutical composition comprising: the compound or the salt thereof, or the solvate thereof according to Claim 1 or 2, and a pharmaceutically acceptable carrier.

4. A pharmaceutical composition comprising: the compound or the salt thereof, or the solvate thereof according to Claim 1 or 2 as an effective component, having both an angiotensin II receptor antagonist activity and a PPARγ activating activity.
